# FASCICULE DE BREVET EUROPEEN

(11) **EP 0 447 478 B1**
(45) Date de publication et mention de la délivrance du brevet: **12.01.1994**
(21) Numéro de dépôt: 90900905.2
(22) Date de dépôt: 30.11.1989
(51) Int. Cl.: A61B 6/00, G01T 1/164

(54) **SYSTEME DE MISE EN PLACE D'UN COLLIMATEUR DANS UNE GAMMA CAMERA**
VORRICHTUNG ZUR AUFSTELLUNG EINES KOLLIMATORS IN EINER GAMMA-KAMERA
SYSTEM FOR INSTALLING A COLLIMATOR IN A GAMMA CAMERA

(30) Priorité: 07.12.1988 FR 8816069
(43) Date de publication de la demande: 25.09.1991
(73) Titulaire: SOPHA MEDICAL, F-75008 Paris (FR)
(72) Inventeur: PARE, Christian, F-78370 Plaisir (FR); FLEURY, Christophe, F-92160 Antony (FR)
(74) Mandataire: Schmit, Christian Norbert Marie
(86) Numéro de dépôt international: FR8900620
(87) Numéro de publication internationale: WO9006083

(56) Documents cités:
- EP-A- 0 156 112
- EP-A- 0 194 728
- JP-A-59 090 073
- JP-A-60 165 568

## Description

La présente invention a pour objet un système de mise en place d'un collimateur dans une gamma caméra. Son utilisation est essentiellement orientée à des fins d'imagerie médicale. Une gamma caméra est l'ensemble des matériels, détecteur statif console, permettant un examen de médecine nucléaire. Le collimateur d'une gamma caméra se fixe sur le détecteur.

On connaît le principe de fonctionnement d'une gamma-caméra. Des patients soumis à un examen sur un tel appareil reçoivent par injections dans leur corps, un traceur contenant un isotope radioactif. Cet isotope se fixe préférentiellement dans un organe particulier selon le type du traceur injecté. La fonction de la gamma caméra est de former l'image de la projection plane de la radioactivité gamma fixée par ledit organe, relevant ainsi son etat fonctionnel. Cette image est obtenue par la détection des rayonnements gamma issus de l'organe examiné et émis dans une seule direction afin de réaliser une projection de la concentration de l'isotope dans l'organe, selon cette direction. Une gamma caméra comprend donc un détecteur de rayonnement gamma, constitué en particulier d'un cristal scintillateur de grande surface recouvert par un réseau de photo-multiplicateurs. Lorsqu'un rayonnement gamma traverse le scintillateur, il y produit une scintillation localisée qui est détecté par plusieurs tubes photo-multiplicateurs situés au voisinage de la scintillation. Les photo-multiplicateurs fournissent un courant proportionnel à la scintillation reçue. Cela permet aussi de localiser l'impact des rayons gamma dans le cristal scintillateur par une évaluation du barycentre des signaux fournis par les photomultiplicateurs.

La sélection des rayonnements gamma détectés émis dans une seule direction, par exemple une direction perpendiculaire à la face d'entrée du scintillateur, est réalisée au moyen d'un dispositif appelé collimateur situé juste en avant et contre le détecteur.

Schématiquement un collimateur est une plaque absorbante, généralement du plomb, percée d'une multitude de conduits qui laissent passer les rayonnements gamma selon un angle solide très réduit, dont l'axe est parallèle à la direction de la projection de l'image de distribution de l'isotope radioactif qu'on veut reproduire.

Dans la pratique les collimateurs sont déterminés en fonction de l'énergie du rayonnement gamma émis par l'isotope injecté au patient et en fonction des caractéristiques de transparence et de résolution spatiale qu'on cherche à obtenir. Grosso-modo, la résolution spatiale et la transparence dépendent de l'ouverture de l'angle solide des conduits du collimateur, et il est clair que ces deux caractéristiques varient en sens inverse. Selon les types d'examen réalisables en médecine nucléaire, on fait appel à des émetteurs de rayons gamma d'énergies différentes, ou bien on exige des caractéristiques de transparence et de résolution particulières pour les collimateurs. Dans ce dernier cas, une gamma caméra est généralement équipée d'une famille de collimateurs présentant des caractéristiques géométriques différentes. En exploitation de routine de la gamma caméra, le collimateur, associé au détecteur de cette caméra pour un examen particulier, doit pouvoir être échangé avec un autre collimateur, à la demande de l'opérateur pratiquant l'examen.

Selon l'énergie du rayonnement à détecter et les caractéristiques d'angle solide recherchées, le collimateur est plus ou moins épais ou, en pratique, plus ou moins lourd. En outre, les détecteurs sont généralement montés en porte à faux sur une potence. Le porte à faux présenté par le détecteur est normalement compensé par des contre poids agencés dans le mécanisme de mobilisation ou de manutention de ce détecteur. Comme ces contre poids ne peuvent pas être facilement modifiés, on a pris l'habitude d'associer aux collimateurs légers (ceux réservés aux rayonnements de faible énergie) des masses périphériques de manière à leur donner un poids égal au poids des collimateurs les plus lourds. Alors que le poids de la partie utile des collimateurs peut varier de 20 à 60 kg, cette technique a conduit à réaliser des collimateurs qui, avec les masses associées, représentent toujours des poids de l'ordre de 60 kg.

La manipulation de collimateurs d'un tel poids ne va pas sans un équipement mécanique. En pratique le détecteur de la gamma caméra est contenu dans une enveloppe métallique en forme de grosse marmite pourvue d'une feuillure pour recevoir le collimateur. Le collimateur forme en quelque sorte le couvercle du détecteur et de l'enveloppe. Le principe de la mise en place consiste à venir soulever le collimateur en s'appuyant par en dessous, sur la face extérieure de ce collimateur, lorsque cette face est tournée vers le bas. Dans de telles manipulations on applique le collimateur au détecteur par en dessous, en ayant au préalable retourné le détecteur. Par exemple, deux bras de manutention montés sur un chariot viennent s'appliquer sous cette face extérieure et dégagent le collimateur de l'endroit où il était stocké. Le chariot, est ensuite déplacé jusqu'à proximité de la gamma caméra. Pour la mise en place du collimateur, comme le détecteur est retourné, l'ouverture de l'enveloppe est vers le bas, et son "couvercle" lui est appliqué en relevant les deux bras de manipulation. Comme cette manipulation est faite par en dessous,elle est exécutée en aveugle.

Le collimateur se présente comme un plateau relativement épais. Dans le cas où les détecteurs sont à champ rond, le collimateur est bien entendu rond. Pour ces collimateurs ronds une solution de fixation est la suivante. Dans le chant de la plaque du collimateur est pratiquée une rainure circulaire qui entoure cette plaque. Au moment de la mise en place du collimateur pour fermer l'enveloppe, cette rainure vient se placer en face d'un système à vis solidaire de l'enveloppe. Le vissage, manuel, de ces vis, permet l'engagement des têtes de ces vis dans la rainure. Une fois que cet engagèment est exécuté, le chariot et les bras de manipulation peuvent être écartés.

Etant donné qu'une gamma caméra doit être utilisée avec toutes les orientations possibles dans l'espace, et notamment se trouver au dessus du patient, les manipulateurs chargés de la mise en place des collimateurs prennent l'habitude de serrer très fort les vis de fixation du collimateur. Ceci permet d'éviter les risques de décrochement de ce collimateur qui, en tombant, pourrait blesser grièvement le patient. Cette technique de montage présente trois inconvénients. Premièrement le système est peu maniable parce qu'on travaille en aveugle au moment de la mise en place du collimateur. En effet, étant donné que la mise en place se fait par en dessous, le manipulateur ne voit pas exactement à quel moment le chariot se trouve bien placé et donc le centrage du collimateur sur l'ouverture de l'enveloppe est peu aisé. Deuxièmement, le serrage trop fort des vis entraîne, au moment du remplacement du collimateur, une action quelquefois délicate de desserrage.

En outre, l'utilisation des gamma caméras à champ d'examen rond tend à disparaître au profit des gamma caméras dont le champ d'examen est de type carré ou rectangulaire. Dans ces conditions le centrage devient encore plus difficile du fait que, pour des raisons mécaniques, le collimateur, lui aussi rectangulaire, doit être inséré dans une ouverture rectangulaire. Ceci est malaisé quand on le fait en aveugle, par en dessous.

On connaît une deuxième solution applicable elle aussi aux collimateurs pour gamma caméra à champ rond. Dans celles-ci les collimateurs possèdent des poignées escamotables qu'un opérateur suffisamment costaud saisit et approche de l'ouverture, orientée vers le haut cette fois, de l'enveloppe de la gamma caméra. Il descend alors à bout de bras le collimateur sur l'ouverture, et, par une action d'un quart de tour, il engage des protubérances montées sur les chants du collimateur ou de l'enveloppe, dans des baïonnettes montées sur la partie correspondante de l'enveloppe ou du collimateur. Ce mode de fixation n'est bien entendu praticable que pour les collimateurs légers, et n'est malheureusement utilisable, à cause du quart de tour, que sur les collimateurs ronds.

On connaît une troisième solution, dans laquelle un chariot saisit un collimateur par des crochets et vient le placer, par au dessus, sur une enveloppe de gamma caméra. Une fois que le collimateur a été placé au dessus de l'enveloppe, des vis sont engagées, au travers du collimateur, de manière à venir se ficher dans une partie massive de l'enveloppe. Les têtes des vis permettent de retenir le collimateur en place lorsqu'on retourne la gamma caméra et que le collimateur a sa face extérieure orientée vers le sol. L'inconvénient présenté par cette technique est lié en grande partie à la nécessité de devoir visser un nombre important de vis : ceci fait perdre du temps aux opérateurs. En outre ces vis peuvent être, comme les précédentes, trop serrées par souci de sécurité, de sorte que les dévissages ultérieurs peuvent devenir difficiles. Dans une demande de brevet EP-A-0 194 728, ce système de vis est remplacé, pour un collimateur à champ rond, par le déplacement en rotation d'une baïonnette. Cependant ce dispositif n'est pas adaptable aux détecteurs à champ rectangulaire.

Par ailleurs, un autre problème se pose : il est celui d'assurer le transport du collimateur, de l'armoire où il est rangé jusqu'à la gamma caméra qui est censée le recevoir, avec suffisamment de sécurité pour que ce collimateur ne puisse pas échapper des bras manipulateurs du chariot.

L'invention a pour objet de remédier à tous ces inconvénients en proposant un système de mise en place du collimateur dans lequel il existe une coopération mécanique entre le chariot manipulateur, le collimateur, et l'enveloppe de la gamma caméra. En particulier le déverrouillage des collimateurs de leur emplacement dans l'armoire de rangement est concomitant avec le verrouillage des collimateurs sur le chariot mobile censé les transporter. De même leur déverrouillage du chariot mobile se produit en même temps que leur verrouillage sur l'enveloppe de la gamma caméra à l'emplacement final de leur utilisation.

En définitive dans l'invention on assure un verrouillage constant des collimateurs sur tous leur parcours entre l'armoire où ils sont stockés et la gamma caméra où ils sont utilisés. L'invention empêche ainsi toute manipulation hasardeuse et tout risque de chute desdits collimateurs durant leur manipulation. Par ailleurs, bien entendu, l'invention simplifie au maximum les opérations de déverrouillage et de verrouillage des collimateurs de leur lieu de départ à leur lieu d'arrivée par une inter-action semi-automatique du chariot avec les mécanismes de verrouillage existant sur l'armoire et/ou sur l'enveloppe de la gamma caméra.

L'invention a donc pour objet un système de mise en place d'un collimateur sur un détecteur à champ rectangulaire d'une gamma caméra, ce système comportant un chariot de manutention, un collimateur, et un détecteur de gamma caméra porté par une enveloppe, caractérisé en ce que
- le chariot comporte un châssis muni de mâchoires mobiles pour saisir le collimateur,
- le collimateur et l'enveloppe comportent un système d'ergots rétractables et d'organes de réaction en coopération avec les ergots, pour retenir le collimateur sur l'enveloppe,
- les mâchoires coopèrent au moment de leur fermeture avec les ergots pour détacher le collimateur de l'enveloppe en même temps qu'elles saisissent le collimateur.

L'invention sera mieux comprise à la lecture de la description qui suit et à l'examen des figures qui l'accompagnent. Celles-ci ne sont données qu'à titre indicatif et nullement limitatif de l'invention. Les figures montrent :
- figure 1 : une représentation schématique du système de l'invention au moment de la mise en place du collimateur sur l'enveloppe ;
- figure 2 : une représentation schématique en coupe du système de montage du collimateur de l'invention sur une gamma caméra.

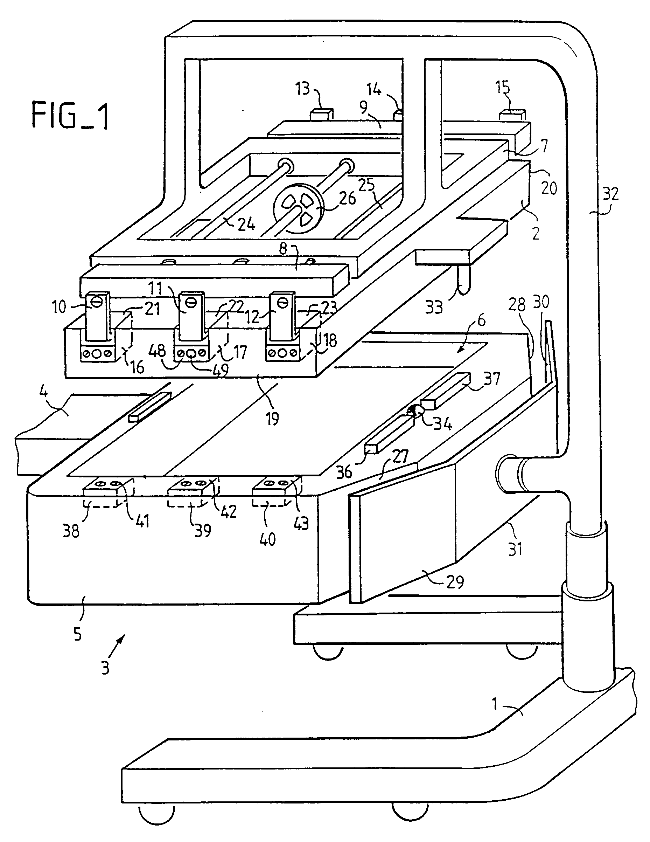

Bien que la description va être faite en référence au montage d'un collimateur sur une gamma caméra on comprendra que des dispositifs semblables peuvent être installés dans l'armoire de rangement, de telle sorte que le chariot puisse effectuer dans cette armoire l'inverse des opérations qu'il effectue sur le collimateur. On va décrire l'opération de montage, l'opération de démontage est simplement déduite en effectuant les opérations en sens inverse. La figure 1 montre un chariot 1 permettant d'approcher un collimateur 2 d'une gamma caméra 3. Le chariot est un chariot à roulettes roulant sur le sol, la gamma caméra est tenue en porte à faux par l'extrémité 4 d'une potence. La gamma caméra 3 comporte une enveloppe 5 possédant une cavité 6 , censée contenir le scintillateur et les tubes photomultiplicateurs. La cavité 6 doit être bouchée par le collimateur 2. Le chariot 1 comporte un châssis 7 muni de mâchoires 8 et 9. Chaque mâchoire comporte, dans l'exemple représenté, trois dents notées respectivement 10 à 12 et 13 à 15. Les dents ont la forme d'une lettre L et possèdent chacune une extrémité recourbée destinée à s'engager dans des cavités telles que 16 à 18 pratiquées dans des chants 19 et 20 en vis à vis du collimateur.

De manière à ce que les extrémités des dents 10 à 15 puissent agripper le collimateur, les cavités telles que 16 à 18 sont obturées, sur une partie supérieure, par des plaques telles que 21 à 23 fixées par tous moyens, par exemple par vissage. Les deux mâchoires sont reliées entre elles et au châssis par des coulisses telles que 24 et 25. Ces coulisses autorisent le déplacement des mâchoires parallèlement au plan du châssis 7. Pour exécuter ce déplacement, une poignée de manoeuvre 26 de type quart de tour, permet , selon le sens de la rotation qu'on lui fait subir, d'éloigner ou d'approcher symétriquement les mâchoires 8 et 9 près de deux longerons latéraux en regard sur le châssis 7. Les mâchoires sont parallèles aux longerons. Lorsque les mâchoires sont approchées par translation l'une de l'autre, les extrémités en L des dents 10 à 15 s'engagent en dessous des plaques 21 à 23 dans les cavités telles que 16 à 18 du collimateur 2. Dans ces conditions le collimateur est maintenu et il peut être déplacé dans la salle d'examen médical pour être approché de la gamma caméra.

L'invention est plus particulièrement avantageuse dans le cas d'utilisation de collimateurs de type rectangulaire auquel cas les mâchoires 8 et 9 sont formées de deux barres droites destinées à se placer de part et d'autre de deux chants opposés du collimateur rectangulaire. Pour la mise en place aisée et un centrage facilité de ce collimateur rectangulaire, l'enveloppe de la gamma caméra est munie en outre d'une face troncônique comportant deux pans inclinés 27 et 28 destinés à venir recevoir en butée des ailes respectivement 29 et 30 d'un bouclier 31 monté sur le chariot 1. Le bouclier peut être réglé une fois pour toutes pour la gamma caméra qu'il est destiné à équiper, de manière à ce que la mise en place des collimateurs soit immédiate.

Le châssis 7 est soutenu par une potence 32. De préférence la potence 32 est télescopique pour autoriser la mise en place par descente du collimateur 2 sur l'enveloppe 5 et pour autoriser par ailleurs le rangement des différents collimateurs sur différentes étagères d'une même armoire de rangement. Autant le bouclier 31 assure le réglage grossier de la mise en place du collimateur, autant un jeu de pions, tels que le pions 33, de forme coniques assurent la mise en place fine du collimateur au moment où ils s'engagent dans des cavités 34 destinées à les recevoir, et pratiquées dans l'enveloppe 5. Ces cavités 34 sont par ailleurs entourées de patins tels que 36 37 servant de pièces d'usure d'une part, et de butée réglable d'autre part, pour régler une machine en fonction de son jeu de quatre ou cinq collimateurs. En effet, les collimateurs sont généralement moulés et ne sont pas toujours aux mêmes cotes. Ils acceptent en général une tolérance de 5/10 ème de millimètre.

En face de l'endroit où se trouvent les cavités 16 à 18 du collimateur, lorsque celui-ci est placé sur l'enveloppe 5, se trouvent des cavités, telles que respectivement 38 à 40, pratiquées dans les chants intérieurs verticaux de cette enveloppe 5 ou d'une feuillure de celle-ci. Ces cavités 38 à 40 sont recouvertes chacune d'une plaque de réaction, respectivement 41 à 43, fixée à l'enveloppe 5 par tous moyens, notamment par vissage.

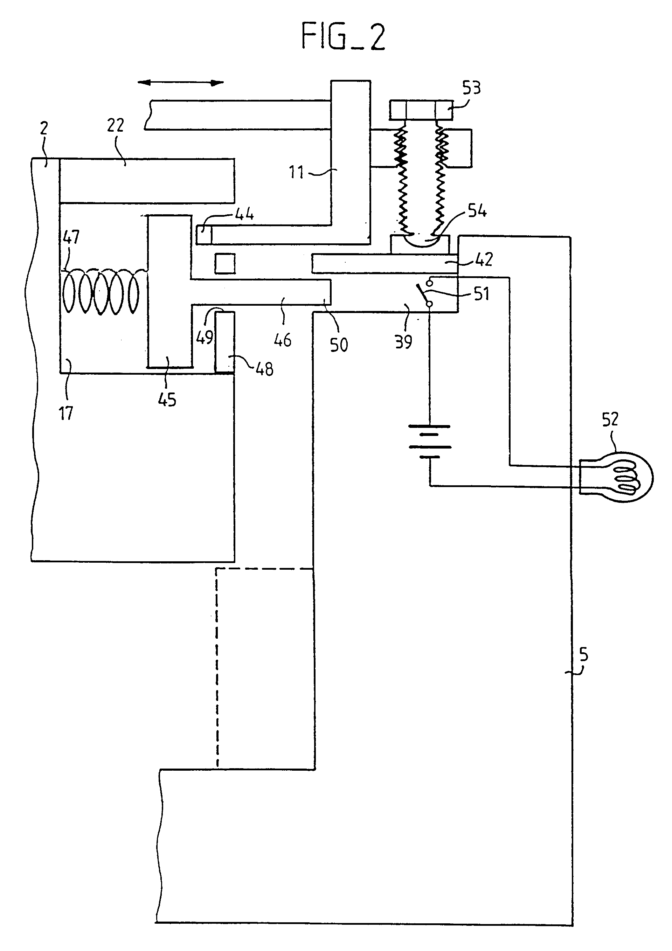

La figure 2 montre, selon le plan de coupe C de la figure 1, le système d'engagement et de désengagement des dents des mâchoires dans les cavités 16 à 18. Sur cette figure, on voit que la dent 11 peut se déplacer, horizontalement, pour entrer ou ressortir de la cavité 17. La cavité 17 est obturée supérieurement par la plaque de réaction 22 qui permet à la dent 11 de saisir le collimateur 2. L'extrémité 44 de la dent 11, au moment de son insertion est par ailleurs en appui sur l'embase 45 d'un ergot 46. L'ergot en forme de tige ronde est autrement normalement repoussé vers l'extérieur de la cavité 17 au moyen d'un ressort 47 qui appuie, d'une part sur le fond de la cavité et d'autre part sur l'arrière de l'embase 45. L'ergot 46 est empêché de sortir de la cavité 17, lorsque la dent 11 est retirée, par la présence d'une plaque de fermeture 48 munie d'un trou 49. Le trou permet le passage de la tige de l'ergot. Ces détails sont également visibles pour la cavité 17 sur la figure 1.

Pendant le transport, la dent 11 est complètement engagée à l'intérieur de la cavité 17 et l'ergot 46 est complètement rétracté à l'intérieur de cette même cavité. Ainsi le collimateur est maintenu par les mâchoires sur le chariot. Lorsque le collimateur repose au moment de sa mise en place sur l'enveloppe, sur les butées 36 et 37, la dent 11 peut être reculée permettant à la tête 50 de l'ergot l'ergot 46 de pénétrer à l'intérieur de la cavité 39 pratiquée en regard dans l'enveloppe 5. Ces deux actions sont ainsi simultanées de sorte que le déverrouillage des mâchoires 8 et 9 entraîne le verrouillage du collimateur sur l'enveloppe 5. En effet, une fois que les ergots tels que 46 sont entrés dans les cavités telles que 39, ils appuient par leur génératrice ,le moment venu, sur les plaques de réaction 42 qui obturent superficiellement ces cavités telles que 39.

Dans ces conditions le collimateur est maintenu sur l'enveloppe.

Au lieu d'une translation longitudinale d'ergots allongée, il est possible d'envisager des ergots courbes qui s'engagent dans l'enveloppe par rotation. Par exemple la tête 44 de la dent 11 peut en appuyant sur le bord de l'embase 45 provoquer une telle rotation. Par ailleurs on pourrait aussi concevoir que les ergots sont au repos dans les cavités 39 de l'enveloppe et que l'arrière de la dent, au moment ou celle-ci se dégage de la cavité 11, provoque (par exemple par rotation) la sortie de ces autres ergots qui viennent s'engager dans ces cavités en regard dans le collimateur. Pour parfaire la sécurité de mise en place, au moment où il pénètre à l'intérieur des cavités 39, les extrémités 50 des ergots appuient sur des interrupteurs tels que 51 qui déclenchent un système de sécurité ou d'alarme 52. Le système 52 présenté est un système à sécurité positive : quel que soit le défaut constaté, y compris un défaut du système 52 lui-même, un témoin de fonctionnement l'indique.

D'une manière complémentaire à la présence des butées 37, la dent 11 peut comporter en partie arrière, une vis de réglage 53 destinée à régler en hauteur la position des dents au moment de l'opération de verrouillage/déverrouillage sur la gamma caméra. En effet, ces opérations doivent être pratiquées avec une gamma caméra sensiblement horizontale. De même un sol sur lequel roule le chariot 1 peut être accidenté et provoquer des défauts de présentation du collimateur dans l'enveloppe. Dans ce but les coulisses 24 et 25 sont plutôt lâches et permettent au collimateur 2 de flotter légèrement autour d'une articulation passant sensiblement par l'axe de la poignée quart de tour 26. Les vis 53 peuvent alors par leur tête 54 venir en appui sur le dessus des plaques de réaction 42 de manière à adapter la hauteur de l'extrémité 44 des dents 11 pour que les extrémités de ces dents pénètrent à l'intérieur des cavités 17 concernées. Dans ces conditions les vis 53 sont de préférence réalisées qu'en regard des dents 11 et 14 centrales. dans le but de disposer de telles dent centrales le nombre des dents sur chaque côté est de préférence un nombre impairs. Pour permettre leur engagement, et aussi pour limiter les jeux, les ergots 46 latéraux ont de préférence un profil conique.

## Revendications

1. Système de mise en place d'un collimateur (2) sur un détecteur à champ rectangulaire d'une gamma caméra (3), ce système comportant un chariot (1) de manutention, un collimateur, et un détecteur de gamma caméra porté par une enveloppe (5), caractérisé en ce que
- le chariot comporte un châssis (7) muni de mâchoires (8,9) mobiles pour saisir le collimateur,
- le collimateur et l'enveloppe comportent un système d'ergots (46) rétractables (47) et d'organes (42) de réaction en coopération avec les ergots, pour retenir le collimateur sur l'enveloppe,
- les mâchoires coopèrent (44) au moment de leur fermeture avec les ergots pour détacher le collimateur de l'enveloppe, en même temps qu'elles saisissent le collimateur.

2. Système selon la revendication 1 caractérisé en ce que les ergots (46) sont portés par le collimateur (3), les organes de réaction (38-40) sont portés par l'enveloppe, et en ce que les ergots se rétractent dans les chants (19,20) du collimateur.

3. Système selon la revendication 1 ou la revendication 2 caractérisé en ce que le système d'ergots comporte un nombre d'ergots double d'un nombre impair.

4. Système selon l'une quelconque des revendications 1 à 3 caractérisé en ce que les ergots ont un profit conique pour limiter les jeux.

5. Système selon l'une quelconque des revendications 1 à 4 caractérisé en ce que les ergots sont rétractables en translation.

6. Système selon l'une quelconque des revendications 1 à 5 caractérisé en ce que les ergots coopèrent avec des interrupteurs (51) électriques reliés à un dispositif (32) de sécurité ou d'alarme.

7. Système selon l'une quelconque des revendications 1 à 6 caractérisé en ce que le chariot comporte un châssis (7) de maintien des deux mâchoires, ce châssis comportant des moyens (26) pour déplacer symétriquement les deux mâchoires par rapport au châssis.

8. Système selon l'une quelconque des revendications 1 à 7 caractérisé en ce que l'enveloppe comporte une face troncônique (27,28) pour s'ajuster dans un bouclier (31) du chariot de manière à automatiser le centrage du collimateur sur l'enveloppe.

9. Système selon l'une quelconque des revendications 1 à 8 caractérisé en ce que le collimateur comporte des pions (33,34) de centrage fin du collimateur sur l'enveloppe.

## Claims

1. System for installing a collimator (2) in a detector with a rectangular field in a gamma camera (3), this system comprising a handling carriage (1), a collimator and a gamma camera detector borne by a casing (5), characterised by the fact that
the carriage comprises a frame (7) equipped with movable jaws (8, 9) to grip the collimator,
the collimator and the casing comprise a system of lugs (46) which are retractable (47) and reaction elements (42) interacting with the lugs, serving to secure the collimator on the case,
the jaws (44) interact at the moment of their closure with the lugs in order to detach the collimator from the casing, at the same time as they grip the collimator.

2. System in accordance with claim 1, characterised by the fact that the lugs (46) are borne by the collimator (3), the reaction elements (38-40) are borne by the casing and the lugs retract in the edges (19, 20) of the collimator.

3. System in accordance with claim 1 or claim 2, characterised by the fact that the system of lugs comprises an odd number of double lugs.

4. System in accordance with any one of claims 1 to 3, characterised by the fact that the lugs have a conical profile in order to limit the play.

5. System in accordance with any one of claims 1 to 4, characterised by the fact that the lugs are retractable in a traversing direction.

6. System in accordance with any one of claims 1 to 5, characterised by the fact that the lugs interact with electrical switches (51) connected to a safety or alarm device (32).

7. System in accordance with any one of claims 1 to 6, characterised by the fact that the carriage comprises a securing frame (7) for the two jaws, this frame comprising means (26) for the symmetrical movement of the two jaws in relation to the frame.

8. System in accordance with any one of claims 1 to 7, characterised by the fact that the casing comprises a face of the shape of a truncated cone (27, 28) designed to fit into a shield (31) of the carriage, in such a way as to render the centering of the collimator on the casing automatic.

9. System in accordance with any one of claims 1 to 8, characterised by the fact that the collimator comprises fine centering pieces (33, 34) for the collimator on the casing.

## Patentansprüche

1. Vorrichtung zum Anbringen eines Kollimators (2) an einem ein rechteckiges Feld aufweisenden Detektor einer Gammakamera (3), wobei die Vorrichtung einen Handhabungswagen (1), einen Kollimator und einen von einem Behältnis (5) getragenen Gammakmera-Detektor aufweist, dadurch gekennzeichnet, daß
- der Wagen ein Gestell (7) enthält, das mit Backen (8,9) zum Erfassen des Kollimators versehen ist,
- der Kollimator und das Behältnis ein System von zurückziehbaren (47) Nasen (46) und mit den Nasen zusammenwirkenden Gegenstücken (42) enthalten, um den Kollimator auf dem Behältnis zu halten,
- die Backen im Augenblick ihres Schließens mit den Nasen zusammenwirken (44), um den Kollimator von dem Behältnis zu lösen, während sie gleichzeitig den Kollimator erfassen.

2. Vorrichtung nach Anspruch 1, dadurch gekennzeichnet, daß die Nasen (46) von dem Kollimator getragen sind, daß die Gegenstücke (38-40) von dem Behältnis getragen sind und daß die Nasen sich in die Schmalseiten (19,20) des Kollimators zurückziehen.

3. Vorrichtung nach Anspruch 1 oder Anspruch 2, dadurch gekennzeichnet, daß das Nasensystem eine Anzahl von Nasen enthält, die das Doppelte einer ungeraden Zahl ist.

4. Vorrichtung nach einem der Ansprüche 1 bis 3, dadurch gekennzeichnet, daß die Nasen zur Begrenzung des Spiels ein konisches Profil haben.

5. Vorrichtung nach einem der Ansprüche 1 bis 4, dadurch gekennzeichnet, daß die Nasen translatorisch zurückziehbar sind.

6. Vorrichtung nach einem der Ansprüche 1 bis 5, dadurch gekennzeichnet, daß die Nasen mit elektrischen Schaltern (51) zusammenwirken, die mit einer Sicherheits- oder Alarmeinrichtung (32) verbunden sind.

7. Vorrichtung nach einem der Ansprüche 1 bis 6, dadurch gekennzeichnet, daß der Wagen ein Gestell (7) zum Halten der beiden Backen aufweist, wobei das Gestell Mittel (26) zur symmetrischen Verstellung der beiden Backen bezüglich des Gestells aufweist.

8. Vorrichtung nach einem der Ansprüche 1 bis 7, dadurch gekennzeichnet, daß das Behältnis eine abgeschrägte Fläche (27,28) hat, um sich in einen Schild (31) des Wagens einzupassen, derart, daß die Zentrierung des Kollimators auf dem Behältnis automatisiert wird.

9. Vorrichtung nach einem der Ansprüche 1 bis 8, dadurch gekennzeichnet, daß der Kollimator Stifte (33,34) zur Feinzentrierung des Kollimators auf dem Behältnis aufweist.
